(19) Europäisches Patentamt
European Patent Office
Office européen des brevets

(11) Publication number: **0 013 878**
**A1**

(12) # EUROPEAN PATENT APPLICATION

(21) Application number: **80100019.1**

(22) Date of filing: **04.01.80**

(51) Int. Cl.³: **C 07 D 209/08**, C 07 D 401/12, C 07 D 401/14, C 07 D 403/12, C 07 D 209/42, A 61 K 31/40

(30) Priority: **18.01.79 CH 491/79**
**18.01.79 CH 496/79**

(43) Date of publication of application: **06.08.80**
**Bulletin 80/16**

(84) Designated Contracting States: **AT BE CH DE FR GB IT LU NL SE**

(71) Applicant: **SANDOZ AG, Lichtstrasse 35, CH-4002 Basel (CH)**

(72) Inventor: **Berthold, Richard, Dr., Ahornstrasse 9, CH-4103 Bottmingen (CH)**

(54) **3-Aminopropoxyaryl derivatives, their preparation and pharmaceutical compositions containing them.**

(57) The compounds of formula I

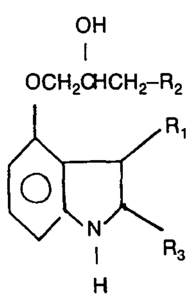

I

where $R_1$, $R_2$ and $R_3$ have various significances, are useful as antiarrhythmic, α-adrenoceptor blocking and, when a cyano or carbamoyl group is attached in the 2 position of the indole nucleus, additionally as β-adrenoceptor blocking agents.

They are prepared by amination.

EP 0 013 878 A1

0013878

Case 100-5147/X

# 3-AMINOPROPOXYARYL DERIVATIVES, THEIR PREPARATION AND PHARMACEUTICAL COMPOSITIONS CONTAINING THEM

The present invention relates to 3-aminopropoxy-aryl derivatives, their preparation and pharmaceutical compositions containing them.

In accordance with the invention there are provided compounds of formula I,

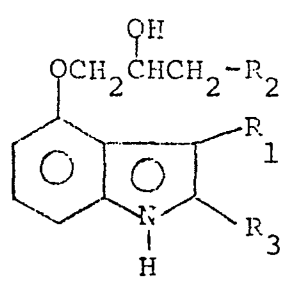

I

wherein

$R_1$ is hydrogen or methyl,

$R_3$ is hydrogen, methyl, hydroxymethyl, carboxyl, alkoxycarbonyl of 2 to 5 carbon atoms, carbamoyl or cyano and

$R_2$ is a group a) or b), groups a) and b) having the following significances:

a) 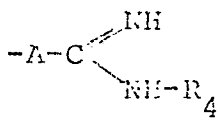 , wherein

A is a group 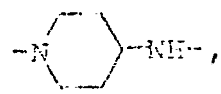 , 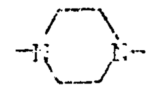 or

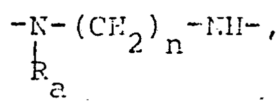 ,

wherein

$R_a$ is hydrogen or alkyl of 1 to 4 carbon atoms and

n is 2, 3 or 4 and

$R_4$ is hydrogen, alkyl of 1 to 4 carbon atoms, alkanoyl of 2 to 4 carbon atoms or benzoyl, and

b) 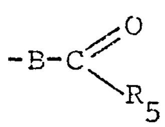 , wherein

B is a group 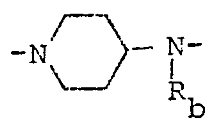 , 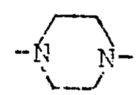 or

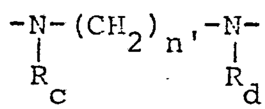 ,

wherein

$R_b$ is hydrogen, alkyl of 1 to 4 carbon atoms, phenyl or phenyl mono- or independently disubstituted by alkyl of 1 to 4 carbon atoms, alkoxy of 1 to 4 carbon atoms or halogen of atomic number of from 9 to 35,

$R_c$ is hydrogen or alkyl of 1 to 4 carbon atoms,

$R_d$ is hydrogen, alkyl of 1 to 4 carbon atoms, phenyl or phenyl mono- or independently disubstituted by alkyl of 1 to 4 carbon atoms, alkoxy of 1 to 4 carbon atoms or halogen of atomic number of from 9 to 35, and

n' is 2, 3 or 4 and

$R_5$ is alkyl of 1 to 4 carbon atoms, furan, phenyl, phenyl mono- or independently disubstituted by alkyl of 1 to 4 carbon atoms, alkoxy of 1 to 4 carbon atoms or halogen of atomic number of from 9 to 35, or is $-NR_eR_f$, wherein

$R_e$ and $R_f$ independently are hydrogen, alkyl of 1 to 4 carbon atoms, phenyl or phenyl mono- or independently disubstituted by alkyl of 1 to 4 carbon atoms, alkoxy of 1 to 4 carbon atoms or halogen of atomic number of from 9 to 35,

and physiologically acceptable hydrolyzable derivatives thereof having the hydroxy group in the 2 position of the 3-aminopropoxy side chain in esterified form.

A group of compounds of formula I are the compounds of formula Ip,

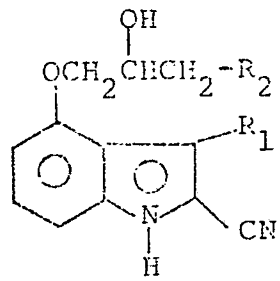

Ip

wherein

$R_1$ and $R_2$ are as defined above.

Another group of compounds of formula I are the compounds of formula Ip'

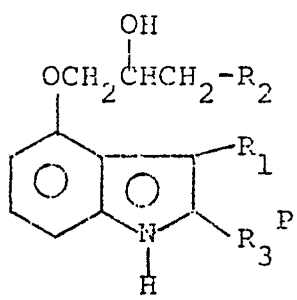

Ip'

wherein

$R_1$ and $R_2$ are as defined above and

$R_3p$ is hydrogen, methyl, hydroxymethyl, carboxyl, alkoxycarbonyl of 2 to 5 carbon atoms or carbamoyl.

Another group of compounds of formula I are the compounds of formula Ia

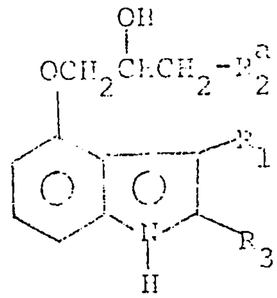

Ia

wherein

$R_1$ and $R_3$ are as defined above and

$R_2^a$ is a group a), as defined above, or a group b') having the significance 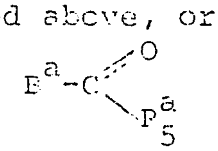 , wherein

$B^a$ and $R_5^a$ are as indicated above for B and $R_5$, with the proviso that, when one of $R_b$ and $R_5$ is alkyl of 1 to 4 carbon atoms, phenyl or phenyl mono- or independently disubstituted by alkyl of 1 to 4 carbon atoms, alkoxy of 1 to 4 carbon atoms or halogen of atomic number of from 9 to 35, the other of $R_b$ and $R_5$ is other than alkyl of 1 to 4 carbon atoms, phenyl or phenyl mono- or independently disubstituted by alkyl of 1 to 4 carbon atoms, alkoxy of 1 to 4 carbon atoms or halogen of atomic number of from 9 to 35,

and physiologically acceptable hydrolyzable derivatives thereof having the hydroxy group in the 2 position of the 3-aminopropoxy side chain in esterified form.

Another group of compounds of formula I are the compounds of formula Ib

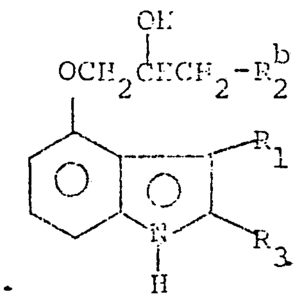

Ib

wherein

$R_1$ and $R_3$ are as defined above and

$R_2^b$ is a group b'') having the significance

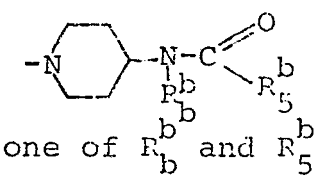

, wherein

one of $R_b^b$ and $R_5^b$ is alkyl of 1 to 4 carbon atoms and the other is phenyl or phenyl mono- or independently disubstituted by alkyl of 1 to 4 carbon atoms, alkoxy of 1 to 4 carbon atoms or halogen of atomic number of from 9 to 35

A group of compounds of formula Ib are the compounds of formula Iba

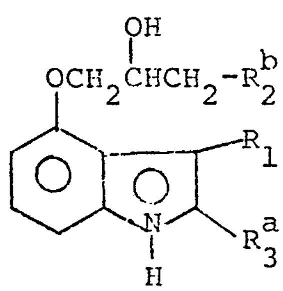

Iba

wherein

$R_1$ and $R_2^b$ are as defined above and

$R_3^a$ is hydroxymethyl, carbamoyl or cyano.

A further group of compounds are physiologically acceptable hydrolyzable derivatives of compounds of formula Ib having the hydroxy group in the 2 position of the 3-aminopropoxy side chain in esterified form.

Physiologically hydrolyzable derivatives are those derivatives which under physiological conditions are split to the corresponding compounds having a hydroxy group in the 2 position of the 3-aminopropoxy side chain.

A group of derivatives in esterified form of the compounds of formula I are e.g. the compounds of formula E

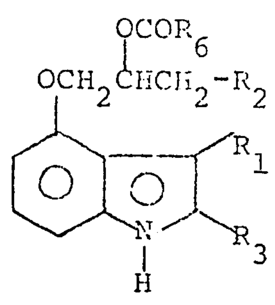

E

wherein

$R_1$ to $R_3$ are as defined above, and

$R_6$ is alkyl of 1 to 12 carbon atoms, cycloalkyl of 3 to 7 carbon atoms, phenyl, phenylalkyl of 7 to 12 carbon atoms, phenyl or phenylalkyl of 7 to 12

BAD ORIGINAL

carbon atoms monosubstituted in the phenyl ring by alkyl of 1 to 4 carbon atoms, or mono- or independently disubstituted in the phenyl ring by halogen of atomic number of from 9 to 35, or mono- or independently di- or independently trisubstituted in the phenyl ring by alkoxy of 1 to 4 carbon atoms.

Groups of derivatives in esterified form of the compounds of formula Ia and Ib are the corresponding derivatives, wherein $R_6$ has the significance indicated above.

Preferred are the compounds wherein the hydroxy group in the 2 position of the 3-aminopropoxy side chain is in free form.

$R_1$ preferably is hydrogen. $R_3$ preferably is carbamoyl, carboxyl or cyano, especially cyano.

$R_2$ preferably is a group b). A preferably is a group 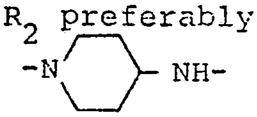 or $-\underset{R_a}{N}-(CH_2)_n-NH-$, especially 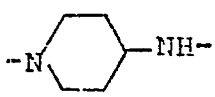. $R_a$ preferably is hydrogen. n preferably is 2. $R_4$ preferably is hydrogen or alkyl, especially alkyl. B preferably is a group 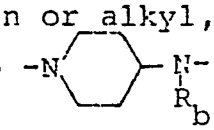 or $-\underset{R_c}{N}-(CH_2)_n,-\underset{R_d}{N}-$, especially 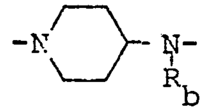. $R_b$ preferably is hydrogen or alkyl, especially hydrogen. $R_c$ preferably is hydrogen. $R_d$ preferably is hydrogen,

BAD ORIGINAL

alkyl or unsubstituted phenyl, especially hydrogen. n' preferably is 2 or 3, especially 2. $R_5$ preferably is alkyl or furan, especially unsubstituted phenyl or $-NR_eR_f$ . $R_e$ preferably is alkyl or phenyl. $R_f$ preferably is hydrogen or alkyl, especially hydrogen. $R_6$ preferably is alkyl or phenyl. Alternatively, $R_6$ conveniently is cycloalkyl, substituted phenyl or substituted or unsubstituted phenylalkyl.

Alkyl (except as indicated herein under for $R_6$ ) and/or alkoxy preferably are of 1 or 2, especially 1 carbon atom. Alkoxycarbonyl or alkanoyl preferably is of 2 or 3, especially 2 carbon atoms. When it is of more than 3 carbon atoms, it is preferably branched in the position α to the carbonyl moiety, as in isopropoxycarbonyl. Halogen preferably is chlorine or bromine, especially chlorine.

When $R_6$ is alkyl, it preferably is of 3 to 5 carbon atoms and preferably is branched, especially in the position α to the carbonyl group to which it is bound, as e.g. in isopropyl, tert-butyl and 3-pentyl, and especially tert-butyl. Cyclcalkyl preferably is of 5 or 6 carbon atoms.

A phenyl ring preferably is unsubstituted. When it is substituted, it preferably is monosubstituted. When it is monosubstituted, the substituent preferably is in the para position. When it is di- or trisubstituted, the substituents preferably are in the meta

BAD ORIGINAL

and para positions. When it is poly-substituted, the substituents preferably are identical. Furan preferably is 2-furan.

In accordance with the invention, a compound of the invention may be obtained by a process comprising reacting a corresponding compound of formula II

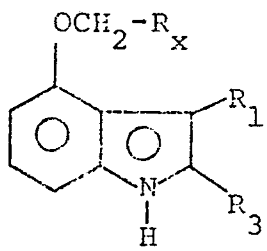

II

wherein $R_1$ and $R_3$ are as defined above, and $R_x$ is a group capable of reacting with a primary or secondary amine to give a 2-amino-1-hydroxyethyl group, with an appropriate compound of formula III

$$R_2-H \qquad III$$

wherein $R_2$ is as defined above, and, where required, appropriately esterifying the 2 position of the 3-aminopropoxy side chain in the resulting compound of formula I.

The amination process may be effected in conventional manner for the production of analogous 3-amino-2-hydroxypropoxyaryl compounds. For example $R_x$ may be a group of formula $-\overset{O}{\widehat{CH-CH_2}}$ or a derivative of this group, e.g. a group of formula $-CH(OH)-CH_2Y$, wherein Y is chlorine, bromine or a group $R_y-SO_2-O-$, wherein $R_y$ is phenyl, tolyl or lower alkyl. Y is

BAD ORIGINAL

especially chlorine. The reaction is preferably effected in isopropanol or in an appropriate ether such as dioxane. Optionally an excess of the amine may be used as solvent. Alternatively, the reaction may be effected in a fusion melt. Suitable reaction temperatures may be from about 20 to about 200°C, conveniently the reflux temperature of the reaction mixture when a solvent is present.

The optional substitution of the 2-hydroxy group in the side chain may be effected in conventional manner. For example, it may be esterified in manner known for the production of analogous esters of 3-amino-2-hydroxypropoxyaryl compounds, if necessary using selective reactions when other reactive groups are present. When $R_3$ is hydroxymethyl or carbamoyl, or when $R_2$ is a group d) or f), such esterification step is effected selectively in the 2 position of the 3-amino-propoxy side chain, conveniently under temporary protection of the other reactive group or groups that may be present, e.g. for hydroxy in the form of e.g. a benzyloxy group, and subsequent selective splitting of the protecting group, e.g. by hydrogenation.

Free forms of the compounds of the invention may be converted into salt forms in conventional manner and vice versa. Suitable acids for acid addition salt formation include maleic, malonic and fumaric acid. When $R_3$ is carboxyl, salts may also be formed with strong bases, e.g. sodium hydroxide.

BAD ORIGINAL

In the compounds of the invention, the carbon atom in e.g. the 2 position of the 3-aminopropoxy side chain is asymmetrically substituted. The compounds may thus exist in the racemic form or in individual optical isomer form. The preferred optical isomer has the S configuration at this asymmetrically substituted carbon atom of the 3-aminopropoxy side chain.

Individual optical isomer forms may be obtained in conventional manner, for example by using optically active starting materials or by fractional crystallisation using optically active acids.

A compound used as a starting material may be obtained in conventional manner.

In particular, a compound of formula II may be obtained by introducing by O-alkylation a group $-OCH_2-R_x$ into a compound of formula IV,

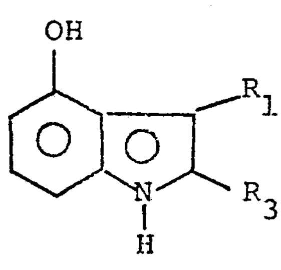

IV

wherein $R_1$ and $R_2$ are as defined above. The compounds of formula IV are preferably reacted in anionic form.

BAD ORIGINAL

4-Hydroxy-1H-indole-2-carbonitrile and 4-Hydroxy-3-methyl-1H-indole-2-carbonitrile may be obtained by splitting off of a water molecule from the corresponding 2-carboxamide derivative, e.g. using titanium tetrachloride.

4-(2,3-Epoxypropoxy)-1H-indole-2-carbonitrile and 4-(2,3-epoxypropoxy)-3-methyl-1H-indole-2-carbonitrile may e.g. also be obtained from the corresponding 2-carboxamide derivative, e.g. using trifluoroacetic acid anhydride.

Insofar as the preparation of any particular starting material is not particularly described, this may be effected in conventional manner.

In the following Examples all temperatures are in degrees Centigrade and are uncorrected.

BAD ORIGINAL

EXAMPLE 1: 4-{2-hydroxy-3-[4-(1-methyl-3-phenyl-ureido)piperidin-1-yl]propoxy}-1H-indole-2-carbonitrile

A mixture of 10 g of 4-(2,3-epoxypropoxy)-1H-indole-2-carbonitrile and 15 g of 1-methyl-3-phenyl-1-(piperidin-4-yl)urea in 150 ml dioxane are heated 20 hours under reflex. The reaction mixture is then cooled , treated with active charcoal and filtered. The solution is concentrated and crystallization induced by the addition of ethanol/HCl (M.P. of hydrochloride of the title compound 241° (dec.) after recrystallization from tetrahydrofuran/methylene chloride).

The starting material is obtained as follows: 7 g 4-(2,3-epoxypropoxy)-1H-indole-2-carboxamide, 90 ml dioxane and 7.2 g pyridine are cooled under stirring to 10°. 10.45 g trifluoroacetic anhydride dissolved in 45 ml dioxane are then slowly added, the temperature being maintained at 10-12°. After 2 hours further stirring at room temperature, 500 ml methylene chloride are added, the solution is agitated and decanted twice with 300 ml water and the organic phase dried over magnesium sulphate. The violet solution is then filtered over talc and the solvent evaporated. The viscous liquid residue is chromatographed over 200 g silica gel (Merck Art. 7733), using methylene chloride having 1 % methanol as an eluant. The pure fractions are dissolved in methylene chloride/methanol, the

BAD ORIGINAL

solution is concentrated and ether added. The crystals formed are filtered off, washed with ether and dried at 60° under vacuum (M.P. of 4-(2,3-epoxypropoxy)-1H-indole-2-carbonitrile: 149-151°).

From the appropriate compound of formula II, wherein $R_x$ is $-CH_2-\underbrace{CH-CH_2}_{O}$ , the following compounds of formula I may be obtained by reaction with the appropriate compound of formula III in analogous manner to Example 1:

BAD ORIGINAL

| Example No. | $R_1$ | $R_3$ | $R_2$ | M.P. |
|---|---|---|---|---|
| 2 | H | CN | 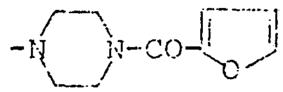 | fn 207-208° |
| 3 | H | CN | 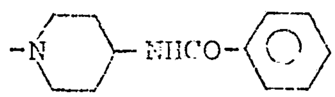 | 203-204° |
| 4 | H | CN | 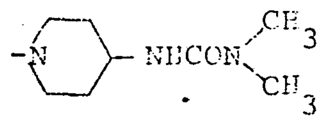 | 179-181° |
| 5 | H | CN | $-NH(CH_2)_2NHCONH-$ 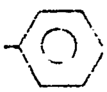 | hmo 146° (dec.) |
| 6 | H | $CONH_2$ | $-NH(CH_2)_2NHCONH-$ 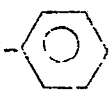 | 105° (dec.) |

fn = bis[base]fumarate

hmo = hydrogen malonate

The compounds of the invention exhibit pharmacological activity in animals.

The compounds exhibit antiarrhythmic activity, as indicated in standard tests. For example, they prolong the functional refractory period in the left guinea pig atrium at a concentration of from $10^{-6}$ to $10^{-4}$ M of the compounds in accordance with the principles of N. Reuter and E. Heeg [Arch.Pharmakol.208 (1971) 323-333].

The compounds are therefore indicated for use as antiarrhythmic agents, e.g. for the treatment of heart rhythm disorders such as heart flutter.

The compounds also exhibit α-adrenoceptor blocking activity, as indicated by standard tests. For example, the inhibition of α-adrenoceptors may be observed in isolated spiral strips of the Vena femoralis of dogs (E. Müller-Schweinitzer and E. Stürmer, Br.J.Pharmacol. [1974] 51, 441-446) at a bath concentration of from about $10^{-7}$ M to about $10^{-5}$ M.

The compounds are therefore indicated for use as α-adrenoceptor blocking agents, e.g. for the prophylaxis and treatment of disorders related to a paralysis of intestine motility, such as paralytic ileus.

BAD ORIGINAL

The compounds having a cyano or carbamoyl group in the 2 position of the indole ring, especially a cyano group, possess β-adrenoceptor blocking activity, as indicated by standard tests. For example, in the isolated, spontaneously-beating guinea pig atrium (method of K. Saameli, Helv.Physiol.Acta 25 [1967] CR 219-CR 221) inhibition of the positive inotropic effect of adrenaline is observed at a bath concentration of about $10^{-9}$ M to about $10^{-6}$ M.

These compounds are therefore indicated for use as β-adrenoceptor blocking agents, e.g. for the prophylaxis and treatment of coronary diseases such as Angina pectoris, of conditions resulting from sympathetic overstimulation, such as nervous heart ailments, of hypertension, of myocardial infarct, for interval migraine treatment, and for the treatment of glaucoma and thyreotoxicosis.

It will be appreciated that it may be necessary to convert a compound having a substituted hydroxy group in the 2 position of the 3-aminopropoxy side chain to

BAD ORIGINAL

the corresponding free hydroxy compound prior to carrying out the tests indicated above for showing α- and β-adrenoceptor blocking activity.

The compounds of formula Ib exhibit more beneficial properties than would be expected for compounds of this type, for example, β-adrenoceptor blockade in the case of the 2-cyano or 2-carbamoyl compounds, especially the 2-cyano compounds, freedom from undesirable side effects, long duration of activity, etc.

For the above-mentioned uses the dosage will, of course, vary depending on the compound employed, mode of administration and treatment desired. However, in general, satisfactory results are obtained when administered at a daily dosage of from about 0.1 mg to about 1000 mg, and dosage forms suitable for oral administration comprise from about 0.25 mg to about 500 mg of the compounds admixed with a solid or liquid pharmaceutical carrier or diluent. Examples of daily doses are from 0.1 to 100 mg .

In general, the 2(S) optical isomers of the compounds are more active than the 2(R) optical isomers as β-adrenoceptor blocking agents.

The compounds may be administered in pharmaceutically acceptable salt form. Such salt forms exhibit the same order of activity as the free forms and are readily prepared in conventional manner. The present

BAD ORIGINAL

invention also provides a pharmaceutical composition comprising a compound of the invention in free form or in pharmaceutically acceptable salt form, in association with a pharmaceutical carrier or diluent. Such compositions may be in the form of, for example, a tablet.

BAD ORIGINAL

## What we claim is:

1. A compound of formula I

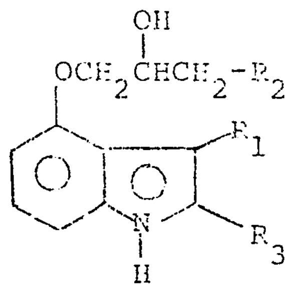

I

wherein

$R_1$ is hydrogen or methyl,

$R_3$ is hydrogen, methyl, hydroxymethyl, carboxyl, alkoxycarbonyl of 2 to 5 carbon atoms, carbamoyl or cyano and

$R_2$ is a group a) or b), groups a) and b) having the following significances:

a) 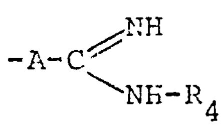 , wherein

A is a group 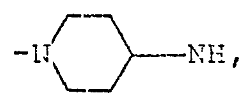 , 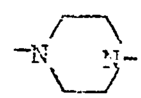 or

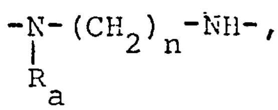

wherein

$R_a$ is hydrogen or alkyl of 1 to 4 carbon atoms and

n is 2, 3 or 4 and

BAD ORIGINAL

$R_4$ is hydrogen, alkyl of 1 to 4 carbon atoms, alkanoyl of 2 to 4 carbon atoms or benzoyl, and

b) 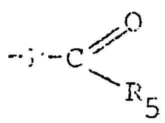 , wherein

B is a group 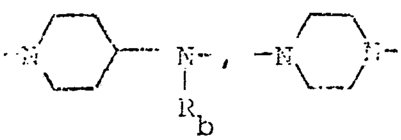 or

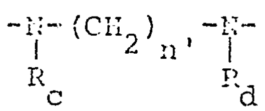 ,

wherein

$R_b$ is hydrogen, alkyl of 1 to 4 carbon atoms, phenyl or phenyl mono- or independently disubstituted by alkyl of 1 to 4 carbon atoms, alkoxy of 1 to 4 carbon atoms or halogen of atomic number of from 9 to 35,

$R_c$ is hydrogen or alkyl of 1 to 4 carbon atoms,

$R_d$ is hydrogen, alkyl of 1 to 4 carbon atoms, phenyl or phenyl mono- or independently disubstituted by alkyl of 1 to 4 carbon atoms, alkoxy of 1 to 4 carbon atoms or halogen of atomic number of from 9 to 35, and

n' is 2, 3 or 4 and

BAD ORIGINAL

$R_5$ is alkyl of 1 to 4 carbon atoms, furan, phenyl, phenyl mono- or independently disubstituted by alkyl of 1 to 4 carbon atoms, alkoxy of 1 to 4 carbon atoms or halogen of atomic number of from 9 to 35, or is $-NR_eR_f$, wherein

$R_e$ and $R_f$ independently are hydrogen, alkyl of 1 to 4 carbon atoms, phenyl or phenyl mono- or independently disubstituted by alkyl of 1 to 4 carbon atoms, alkoxy of 1 to 4 carbon atoms or halogen of atomic number of from 9 to 35,

or a physiologically acceptable hydrolyzable derivative thereof having the hydroxy group in the 2 position of the 3-aminopropoxy side chain in esterified form, or salts thereof.

2. A compound of claim 1 having the formula Ip

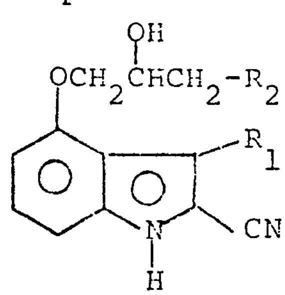

Ip

wherein $R_1$ and $R_2$ are as defined in claim 1, or salts thereof.

BAD ORIGINAL

3. A compound of claim 1 having the formula Ip'

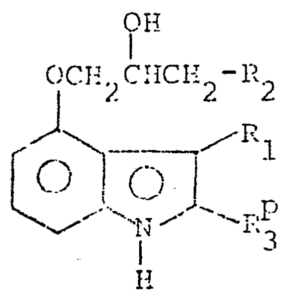

Ip'

wherein

$R_1$ and $R_2$ are as defined in claim 1 and

$R_3^p$ is hydrogen, methyl, hydroxymethyl, carboxyl, alkoxycarbonyl of 2 to 5 carbon atoms or carbamoyl, or salts thereof.

4. A compound of claim 1 having formula Ia

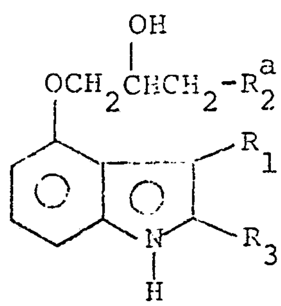

Ia

wherein

$R_1$ and $R_3$ are as defined in claim 1, and

$R_2^a$ is a group a), as defined in claim 1, or a group b') having the significance 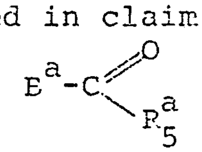 , wherein

$B^a$ and $R_5^a$ are as indicated in claim 1 for B and $R_5$, with the proviso that, when one of $R_b$ and $R_5$

BAD ORIGINAL

is alkyl of 1 to 4 carbon atoms, phenyl or phenyl mono- or independently disubstituted by alkyl of 1 to 4 carbon atoms, alkoxy of 1 to 4 carbon atoms or halogen of atomic number of from 9 to 35, the other of $R_b$ and $R_5$ is other than alkyl of 1 to 4 carbon atoms, phenyl or phenyl mono- or independently disubstituted by alkyl of 1 to 4 carbon atoms, alkoxy of 1 to 4 carbon atoms or halogen of atomic number of from 9 to 35,

or a physiologically acceptable hydrolyzable derivative thereof having the hydroxy group in the 2 position of the 3-aminopropoxy side chain in esterified form,

or salts thereof.

5. A physiologically acceptable hydrolyzable derivative of a compound of claim 1 of formula Ib

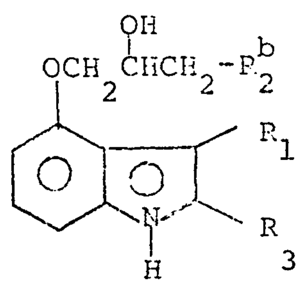

Ib

wherein

$R_1$ and $R_3$ are as defined in claim 1, and

$R_2^b$ is a group b'') having the significance

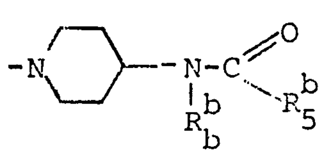

, wherein

BAD ORIGINAL

one of $R_6^b$ and $R_5^b$ is alkyl of 1 to 4 carbon atoms and the other is phenyl or phenyl mono- or independently disubstituted by alkyl of 1 to 4 carbon atoms, alkoxy of 1 to 4 carbon atoms or halogen of atomic number of from 9 to 35,

having the hydroxy group in the 2 position of the 3-aminopropoxy side chain in esterified form,
or salts thereof.

6. A compound of claim 1 having formula Iba

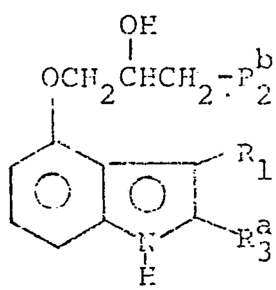

Iba

wherein
$R_1$ is as defined in claim 1,
$R_2^b$ is as defined in claim 5 and
$R_3^a$ is hydroxymethyl, carbamoyl or cyano,
or salts thereof.

7. A compound of claim 1 having formula I as defined in claim 1, wherein $R_2$ is hydrogen, $R_3$ is cyano and

BAD ORIGINAL

either $R_1$ is 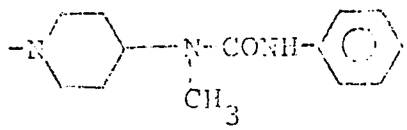

or $R_1$ is 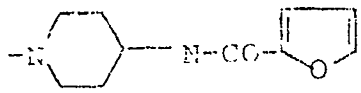

or $R_1$ is 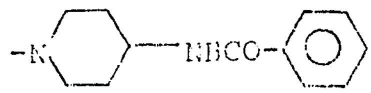

or $R_1$ is 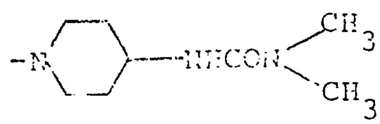

or $R_1$ is 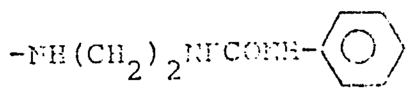

or salts thereof.

8. A compound of claim 1 having formula I as defined in claim 1, wherein $R_2$ is hydrogen, $R_3$ is carbamoyl and $R_1$ is 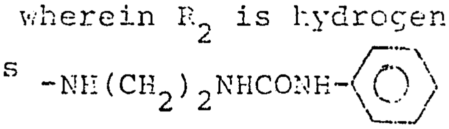

or salts thereof.

9. A process for the production of a compound of claim 1 which comprises reacting a corresponding compound of formula II,

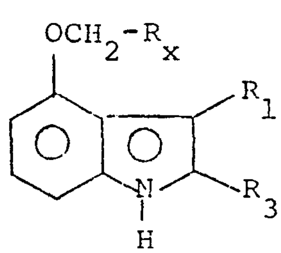

II

BAD ORIGINAL

wherein $R_1$ and $R_3$ are as defined in claim 1, and

$R_x$ is a group capable of reacting with a primary or secondary amine to give a 2-amino-1-hydroxyethyl group,

with an appropriate compound of formula III

$$R_2-H \qquad III$$

wherein $R_2$ is as defined in claim 1, and, where required, appropriately esterifying the 2 position of the 3-aminopropoxy side chain in the resulting compound of formula I.

10. A pharmaceutical composition which comprises a compound of any one of claims 1 to 8 or a pharmaceutically acceptable salt thereof in association with a pharmaceutical carrier or diluent.

3700/VA/GD

BAD ORIGINAL

European Patent Office

# EUROPEAN SEARCH REPORT

0013878

Application number

EP 80 10 00..

| DOCUMENTS CONSIDERED TO BE RELEVANT | | | CLASSIFICATION OF THE APPLICATION (Int. Cl.) |
|---|---|---|---|
| Category | Citation of document with indication, where appropriate, of relevant passages | Relevant to claim | |
| | DE - A - 1 593 901 (I.C.I.)<br>* Abstract *<br>-- | 1-10 | C 07 D 209/08<br>401/12<br>401/14<br>403/12<br>209/..<br>A 61 K 31/40 |
| | FR - A - 1 547 056 (SANDOZ S.A.)<br>* Claims *<br>-- | 1-10 | |
| | CH - A - 469 002 (SANDOZ S.A.)<br>* Page 1 *<br>--- | 1-10 | TECHNICAL FIELDS SEARCHED (Int.Cl.)<br>C 07 D 209/00<br>401/00<br>403/00 |

CATEGORY OF CITED DOCUMENTS

X particularly relevant
A technological background
O non-written disclosure
P intermediate document
T theory or principle underlying the invention
E conflicting application
D document cited in the application
L citation for other reasons

& member of the same patent family,
corresponding document

The present search report has been drawn up for all claims

| Place of search | Date of completion of the search | Examiner |
|---|---|---|
| The Hague | 29-04-1980 | MAISONNEUVE |

EPO Form 1503.1 06.78

BAD ORIGINAL